# EUROPEAN PATENT SPECIFICATION

(11) **EP 2 772 227 B1**
(45) Date of publication and mention of the grant of the patent: **06.09.2017**
(21) Application number: 11874705.4
(22) Date of filing: 15.12.2011
(51) Int. Cl.: A61F 2/24, C08J 3/24, A61L 27/00, B01D 61/36

(54) **METHOD AND DEVICE FOR FIXING ARTIFICIAL BIOPROSTHETIC VALVE BY MEMBRANE PERVAPORATION**
VERFAHREN UND VORRICHTUNG ZUR BEFESTIGUNG EINER KÜNSTLICHEN BIOPROTHETISCHEN HERZKLAPPE MITTELS MEMBRANPERVAPORATION
PROCÉDÉ ET DISPOSITIF DE FIXATION D'UNE BIOPROTHÈSE VALVULAIRE ARTIFICIELLE PAR PERVAPORATION MEMBRANAIRE

(30) Priority: 27.10.2011 CN 201110332309
(43) Date of publication of application: 03.09.2014
(73) Proprietor: Shanghai MicroPort CardioFlow Medtech Co., Ltd., Shanghai 201203 (CN)
(72) Inventor: CHEN, Dakai, Shanghai 201203 (CN); LI, Yu, Shanghai 201203 (CN); DONG, Jiaoming, Shanghai 201203 (CN); FANG, Yuan, Shanghai 201203 (CN); TIAN, Cong, Shanghai 201203 (CN); HUANG, Feng, Shanghai 201203 (CN); CHEN, Guoming, Shanghai 201203 (CN); ZHANG, Xiaoyi, Shanghai 201203 (CN); YUE, Chengyun, Shanghai 201203 (CN); LUO, Qiyi, Shanghai 201203 (CN)
(74) Representative: Dantz, Jan Henning
(86) International application number: PCT/CN2011/084056
(87) International publication number: WO 2013/060067

(56) References cited:
- EP-A1- 1 693 025
- CN-A- 102 172 338
- CN-A- 102 178 571
- CN-Y- 201 244 101
- US-A1- 2010 022 677
- US-A1- 2010 196 870
- ENGELMAYR G C ET AL: "A novel bioreactor for the dynamic flexural stimulation of tissue engineered heart valve biomaterials", BIOMATERIALS, ELSEVIER SCIENCE PUBLISHERS BV., BARKING, GB, vol. 24, no. 14, 1 June 2003 (2003-06-01), pages 2523-2532, XP004419997, ISSN: 0142-9612, DOI: 10.1016/S0142-9612(03)00051-6

## Description

### TECHNICAL FIELD

The present invention relates to a device for fixing an artificial bioprosthetic valve. According to another aspect, the present invention relates to a method for fixing an artificial bioprosthetic valve.

### BACKGROUND ART

Among chemical modifiers for an artificial bioprosthetic valve, glutaraldehyde is a classical fixation cross-linking agent, and it is widely applied to tanning and preservation of various artificial bioprosthetic valve materials. At present, a common method for fixing the artificial bioprosthetic valve is a general chemical solvent method, in which the artificial bioprosthetic valve undergoes a chemical reaction in a liquid condition. However, the glutaraldehyde has a side effect, i.e., free aldehyde groups generated by the glutaraldehyde have a cytotoxic effect, leading to the loss of a large number of tissue matrices with causing calcification of the artificial bioprosthetic valve, changing biomechanical properties thereof, and leading to failure of the artificial bioprosthetic valve after the surgery.

At present, the failure of the artificial bioprosthetic valve caused by the above reasons is mainly alleviated by choosing a more appropriate concentration of glutaraldehyde, processing time and pressure for cross-linking fixation. However, the pressure within the valve tissue during the cross-linking fixation can affect the internal configuration of the artificial bioprosthetic valve and damage the collagen structure, and even cause collapse of the collagen fibers, thereby affecting the service life and durability of the artificial bioprosthetic valve. Thus, a zero-pressure or low-pressure fixation should be adopted as far as possible, and an elastic valve frame should be used to ensure the integrity of the blade tissue of the artificial bioprosthetic valve and reduce the stress injury.

Poor durability is the most important weakness of the artificial bioprosthetic valve. Thus, the essential focus for developing the bioprosthetic valve is to improve the durability of the artificial bioprosthetic valve.

The article "High-concentration glutaraldehyde fixation of bovine pericardium in organic solvent and post-fixation glycine treatment: in vitro material assessment and in vivo anticalcification effect" of Cheul Lee, Soo Hwan Kim, Seung-Hwa Choi, Yong Jin Kim. et al., Eur J Cardiothorac Surg, 2011, 39: 381-387, analyzed the effects of the concentration of glutaraldehyde, the processing time and the pressure for cross-linking fixation on an artificial bioprosthetic valve, and carried out experiments in terms of mechanical properties and anticalcification on the artificial bioprosthetic valve. The general chemical solvent method is adopted in this article.

The article "The vapor pressure of pure and aqueous glutaraldehyde" of James D. Olson, Fluid Phase Equilibria, 1998, 150-151:713-720, analyzed volatilization speeds of glutaraldehyde solutions with different concentrations at different pressures, revealing that the glutaraldehyde has a relatively good volatility. It is proposed in this article that the volatile glutaraldehyde can also chemically modify an artificial bioprosthetic valve. However, the volatilization speed of the glutaraldehyde is not controlled in this article.

The artificial bioprosthetic valves disclosed in the above documents have defects in terms of the durability. Further, according to the general chemical solvent method, as the reaction time increases, an attenuation phenomenon will occur in terms of the concentration of the solution. Furthermore, a processing problem arises due to the residual solution after reaction.

EP 1 693 025 A1 discloses a method of manufacturing a tissue-engineered prosthesis, in particular a heart valve, and a bioreactor for such a method. The bioreactor comprises means for evaporating solvent in the process of fixing the heart valve.

In the article "A novel bioreactor for the dynamic flexural stimulation of tissue engineered heart valve biomaterials" of George C. Engelmayr jr. et. al., biomaterials 24 (2003 2523-2532), the effect of dynamic flexural stimulation on tissue-engineered heart valve biomaterials is studied.

CN 102 172 338 A discloses an artificial biologic valve gradient immersed chemical modification device which comprises a multi-stage reaction solution container, a clamp, a moving device, a rotary hanger, a machine frame and a motor. The device performs multi-stage modification of the artificial biologic valve by using a gradient immersed chemical modification method and realizes the continuous operation of the modification of the artificial biologic valve.

### SUMMARY OF THE INVENTION

In view of the above, the present invention provides a method and a device for fixing an artificial bioprosthetic valve by membrane pervaporation in order to overcome the above defects existing in the prior art.

According to a first aspect, the present invention provides a device for fixing an artificial bioprosthetic valve by membrane pervaporation, comprising a storage tank for storing a reactant liquid, a reaction chamber, a fixation clamp for fixing the artificial bioprosthetic valve, a pervaporation membrane assembly and a vacuum pump. The fixation clamp is positioned inside the reaction chamber; the pervaporation membrane assembly is positioned inside the reaction chamber upstream of the fixation clamp; the storage tank is connected to the reaction chamber upstream of the reaction chamber; and the vacuum pump is connected to the reaction chamber downstream of the reaction chamber. By means of the co-action of the vacuum pump and the pervaporation membrane assembly, the reactant liquid in the storage tank undergoes vaporization, so that the vaporized reactant liquid permeates through the membrane assembly to the artificial bioprosthetic valve on the fixation clamp, thereby the artificial bioprosthetic valve undergoing a chemical modification reaction.

Preferably, the device of the present invention further comprises a frame, the storage tank, the reaction chamber and the vacuum pump each being fixed to the frame, wherein the vacuum pump may be fixed to a bottom of the frame.

According to the present invention, the pervaporation membrane assembly comprises one or more of a flat pervaporation membrane assembly, a hollow fiber pervaporation membrane assembly, and a roll pervaporation membrane assembly.

Preferably, the device of the present invention further comprises: a stirring motor, a stirring rod and a stirring blade, the stirring rod being fixed to an end portion of the stirring motor; and the stirring blade being fixed to the stirring rod. The stirring rod and the stirring blade are driven by the stirring motor to stir the reactant liquid in the storage tank, so that the concentration of the reactant liquid is kept uniform throughout the storage tank. The stirring motor may be fixed to an upper part of the frame.

Preferably, the device of the present invention further comprises a rotating disc and a rotating motor, the rotating disc being fixed to an end portion of the rotating motor and located inside the reaction chamber; and the fixation clamp being fixed to the rotating disc. The rotation speed of the rotating disc is adjusted by the rotating motor, so that the chemical modification reaction is carried out more evenly. The rotating motor may be fixed to a top of the frame.

Preferably, the device of the present invention further comprises heating module assemblies, the heating module assemblies being fixed to peripheries of the storage tank and the reaction chamber, respectively, so as to facilitate vaporization of the reactant liquid.

Preferably, the device of the present invention further comprises a gas-liquid separator and a residue tank, the gas-liquid separator being connected to the reaction chamber downstream of the reaction chamber and in turn connected to the vacuum pump to separate the reactant liquid exiting the reaction chamber from the air for a subsequent processing; and the residue tank being connected to the reaction chamber below the pervaporation membrane assembly to collect a residual solution after reaction.

According to a second aspect, the present invention provides a method for fixing an artificial bioprosthetic valve by membrane pervaporation using the above device, comprising the steps of: fixing one end of the artificial bioprosthetic valve to the fixation clamp of the device for fixing an artificial bioprosthetic valve by membrane pervaporation of the present invention and leaving the other end freely suspended; activating the vacuum pump; activating the pervaporation membrane assembly to allow the vaporized reactant liquid to enter the reaction chamber and permeate through the pervaporation membrane assembly to the artificial bioprosthetic valve; and letting the artificial bioprosthetic valve react in the reaction chamber for a period of time. The reactant liquid comprises a glutaraldehyde solution.

Preferably, the method of the present invention further comprises the step of: activating the stirring motor to drive the stirring rod and the stirring blade to stir the reactant liquid in the storage tank, so that the concentration of the reactant liquid is kept uniform throughout the storage tank.

Preferably, the method of the present invention further comprises the step of: activating the rotating motor. The rotation speed of the rotating disc is adjusted by the rotating motor, so that the chemical modification reaction is carried out more evenly.

Preferably, the method of the present invention further comprises the step of: activating the heating module assembly to facilitate vaporization of the reactant liquid.

Preferably, the process parameter ranges of the method for fixing an artificial bioprosthetic valve by membrane pervaporation according to the present invention are as follows: the permeation rate of the membrane assembly is 20∼ 180 g/(m²· hour); the pressure of the reaction chamber is 0 ∼ 5000 Pa; the concentration of the glutaraldehyde solution is 0.5∼50%; the rotation speed of the stirring rod is 0∼200 rpm; and the rotation speed of the rotating disc is 0∼ 200 rpm.

By means of a membrane pervaporation method, the device and the method of the present invention allow glutaraldehyde monomers to enter the reaction zone of the artificial bioprosthetic valve at a constant rate, and meanwhile, the reaction zone of the artificial bioprosthetic valve is in a low-pressure condition, thereby ensuring that the artificial bioprosthetic valve is located in a reaction zone containing a constant concentration of glutaraldehyde and having a low-pressure condition during the chemical modification process. The device and the method of the present invention not only shorten the chemical modification processing time of the artificial bioprosthetic valve, but also the artificial bioprosthetic valve can advantageously maintain the collagen fiber texture structure of the artificial bioprosthetic valve during the chemical modification process, thereby improving the overall durability of the artificial bioprosthetic valve.

Further, according to the device and the method of the present invention, the artificial bioprosthetic valve undergoes a chemical reaction in a gaseous environment, thus the permeability is better and the rate and efficiency of the reaction are higher during the chemical reaction. Also, according to the general chemical solvent method, as the reaction time increases, an attenuation phenomenon will occur in terms of the concentration of the solution. In contrast, according to the device and the method of the present invention, the properties such as the concentration of the chemical modifier entering the reaction zone are always kept constant while vaporizing the reactant liquid. Furthermore, according to the general chemical solvent method, since the concentration of the solution attenuates as the time goes by, the utilization of the reactant liquid is poor, and a problem arises due to processing of the residual solution after reaction. In contrast, the device and the method of the present invention maximize the utilization of the reactant liquid by vaporizing the reactant liquid, wherein the reactant liquid of 1ml can be vaporized into a reaction area of 10 mm², and the problem arose due to the processing of the residual solution is greatly alleviated. Meanwhile, thanks to the improvement of the permeability of the reaction, the reaction procedure is also greatly expedited.

Furthermore, the method and the device of the present invention are significantly superior to the prior art in terms of the chemical modification of the artificial bioprosthetic valve.

### BRIEF DESCRIPTION OF THE DRAWINGS

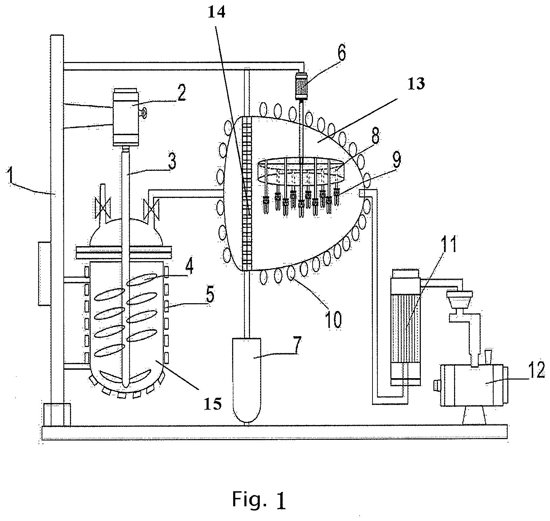

Fig. 1 is a schematic diagram of the structure of the device for fixing an artificial bioprosthetic valve by membrane pervaporation of the present invention.

### List of reference signs

1: frame; 2: stirring motor; 3: stirring rod; 4: stirring blade; 5: heating module assembly; 6: rotating motor; 7: residue tank; 8: rotating disc; 9: fixation clamp; 10: heating module assembly; 11: gas-liquid separator; 12: vacuum pump; 13: reaction chamber; 14: pervaporation membrane assembly; 15: storage tank.

### DETAILED DESCRIPTION

The device and the method of the present invention will be described in detail below with reference to the figure. The embodiment is a further description of the present invention. The embodiment is not intended to limit the scope of the present invention.

For the sake of description, the terms "upstream" and "downstream" are used herein. By these terms, it is meant the flowing direction of the reactant liquid (e.g., glutaraldehyde) of the present invention.

Fig. 1 is a schematic diagram of the structure of the device for fixing an artificial bioprosthetic valve by membrane pervaporation of the present invention. As shown in Fig. 1, the device for fixing an artificial bioprosthetic valve by membrane pervaporation of the present invention comprises a frame 1, a stirring motor 2, a stirring rod 3, a stirring blade 4, heating module assemblies 5 and 10, a rotating motor 6, a residue tank 7, a rotating disc 8, a fixation clamp 9 for fixing the artificial bioprosthetic valve, a gas-liquid separator 11, a vacuum pump 12, a reaction chamber 13, a pervaporation membrane assembly 14 and a storage tank 15 for storing a glutaraldehyde solution. The reaction chamber and the storage tank 15 are both fixed to the frame 1; the storage tank 15 is connected to the reaction chamber 13 upstream of the reaction chamber 13; the stirring motor 2 is fixed to an upper part of the frame 1; the stirring rod 3 is fixed to an end portion of the stirring motor 2; the stirring blade 4 is fixed to the stirring rod 3 and located inside the storage tank 15; the heating module assemblies 5 and 10 are fixed to peripheries of the storage tank 15 and the reaction chamber 13, respectively, to facilitate vaporization of the reactant liquid; the rotating motor 6 is fixed to a top of the frame 1; the rotating disc 8 is fixed to an end portion of the rotating motor 6 and located inside the reaction chamber 13; the fixation clamp 9 is fixed to the rotating disc 8; the pervaporation membrane assembly 14 is positioned inside the reaction chamber 13 upstream of the fixation clamp 9; the residue tank 7 is located below the pervaporation membrane assembly 14 and connected to the reaction chamber 13 to collect the residual solution after reaction; the gas-liquid separator 11 is connected to the reaction chamber 13 downstream of the reaction chamber 13 to separate the reactant liquid exiting the reaction chamber 13 from the air for a further processing; and the vacuum pump 12 is connected to the gas-liquid separator 11 downstream and fixed to a bottom of the frame 1.

The appropriate ranges of the process parameters of the device of the present invention are as follows:

| *Process parameters* | *Numeric ranges* |
|---|---|
| the permeation rate of the membrane assembly | 20∼180 g/(m²·hour) |
| the pressure of the reaction chamber | 0∼5000 Pa |
| the concentration of the glutaraldehyde solution | 0.5∼50% |
| the rotation speed of the stirring rod | 0∼200 rpm |
| the rotation speed of the rotating disc | 0∼200 rpm |

### Example 1: the general chemical solvent method

The artificial bioprosthetic valve was placed in the glutaraldehyde solution of 0.625% for 10 days. The artificial bioprosthetic valve placed in the glutaraldehyde solution was processed by way of swivel oscillation, and the rotation speed was 125 rpm.

### Example 2: the present invention

One end of the artificial bioprosthetic valve was fixed to the fixation clamp 9 of the device for fixing an artificial bioprosthetic valve by membrane pervaporation of the present invention, and the other end was left freely suspended. The stirring rod 3 and the stirring blade 4 were driven by the stirring motor 2 to stir the glutaraldehyde solution of 2% in the storage tank 15, and meanwhile, the vacuum pump 12 was activated so that the pressure of the reaction chamber 13 became 20 Pa. Next, the pervaporation membrane assembly 14 was activated to allow the vaporized glutaraldehyde to enter the reaction chamber 13 and permeate through the membrane assembly 14 to the artificial bioprosthetic valve, and the permeation rate of the membrane assembly was maintained at about 50 g/(m²·hour). The artificial bioprosthetic valve reacted in the reaction chamber 13 for 14 hours, and the rotation speed of the rotating disc 8 was adjusted to 120 rpm by the rotating motor 6 during the reaction process.

Table 1 shows a comparison of mechanical properties of the artificial bioprosthetic valve prepared by adopting the device for fixing an artificial bioprosthetic valve by membrane pervaporation according to the present invention and the artificial bioprosthetic valve prepared by adopting the process of immersing the artificial bioprosthetic valve in the chemical solution according to the prior art. It can be obviously seen from Table 1 that the mechanical property of the artificial bioprosthetic valve prepared by adopting the device for fixing an artificial bioprosthetic valve by membrane pervaporation according to the present invention is greatly improved. Further, the processing procedure of the artificial bioprosthetic valve prepared by adopting the device for fixing an artificial bioprosthetic valve by membrane pervaporation according to the present invention is greatly expedited.

**Table 1**

| *Test method* | *Tensile strength (MPa)* | *Elastic modulus (MPa)* |
|---|---|---|
| The general chemical solvent method | 12.23±1.1 | 46.05±4.6 |
| The present invention | 13.89±0.7 | 92.07±7.5 |

The pervaporation membrane assembly 14 of the present invention comprises one or more of a flat pervaporation membrane assembly, a hollow fiber pervaporation membrane assembly, and a roll pervaporation membrane assembly. The structure of the membrane assembly is well known in the art, and thus no details are given herein.

The device and the method for fixing an artificial bioprosthetic valve by membrane pervaporation of the present invention can allow glutaraldehyde monomers to enter the reaction zone of the artificial bioprosthetic valve at a constant rate, and meanwhile, the reaction zone of the artificial bioprosthetic valve is in a low-pressure condition, so that the artificial bioprosthetic valve can advantageously preserve the collagen fiber texture structure of the artificial bioprosthetic valve during the chemical modification process. The device and the method according to the present invention not only shorten the chemical modification processing time of the artificial bioprosthetic valve, but also can hold the collagen fiber texture structure of the artificial bioprosthetic valve during the chemical modification process, thereby the prepared artificial bioprosthetic valve has a better overall durability.

In the above description, the method and the device of the present invention adopt the glutaraldehyde as the chemical modifier. However, the device and the method of the present invention may also use other chemical modifiers, e.g., formaldehyde and acetaldehyde.

It is appreciated to those skilled in the art that the above description is merely illustrative.

## Claims

1. A device for fixing an artificial bioprosthetic valve by membrane pervaporation, comprising a storage tank (15) for storing a reactant liquid, whereby the reactant liquid is selected from a group consisting of a glutaraldehyde solution, a formaldehyde solution and an acetaldehyde solution, a fixation clamp (9) for fixing the artificial bioprosthetic valve, a reaction chamber (13) and a vacuum pump (12), the fixation clamp (9) being positioned inside the reaction chamber (13), **characterized in that** the device comprises a pervaporation membrane assembly (14) being positioned inside the reaction chamber (13) upstream of the fixation clamp (9), the storage tank (15) being connected to the reaction chamber (13) upstream of the reaction chamber (13) and the vacuum pump (12) being connected to the reaction chamber (13) downstream of the reaction chamber (13).

2. The device for fixing an artificial bioprosthetic valve by membrane pervaporation according to claim 1, **characterized in that** the pervaporation membrane assembly (14) comprises one or more of a flat pervaporation membrane assembly (14), a hollow fiber pervaporation membrane assembly (14), and a roll pervaporation membrane assembly (14).

3. The device for fixing an artificial bioprosthetic valve by membrane pervaporation according to claim 1 or 2, **characterized in that** the device further comprises: a stirring motor (2), a stirring rod (3) and a stirring blade (4), the stirring rod (3) being fixed to an end portion of the stirring motor (2); and the stirring blade (4) being fixed to the stirring rod (3), thereby the stirring rod (3) and the stirring blade (4) being driven by the stirring motor (2) to stir the reactant liquid in the storage tank (15).

4. The device for fixing an artificial bioprosthetic valve by membrane pervaporation according to claim 1 or 2, **characterized in that** the device further comprises a rotating disc (8) and a rotating motor (6), the rotating disc (8) being fixed to an end portion of the rotating motor (6) and located inside the reaction chamber (13); and the fixation clamp (9) being fixed to the rotating disc (8), thereby the rotation speed of the rotating disc (8) being adjusted by the rotating motor (6).

5. The device for fixing an artificial bioprosthetic valve by membrane pervaporation according to claim 1 or 2, **characterized in that** the device further comprises heating module assemblies, the heating module assemblies being fixed to peripheries of the storage tank (15) and the reaction chamber (13), respectively, to facilitate vaporization of the reactant liquid.

6. The device for fixing an artificial bioprosthetic valve by membrane pervaporation according to claim 1 or 2, **characterized in that** the device further comprises a gas-liquid separator and a residue tank, the gas-liquid separator being connected to the reaction chamber (13) downstream of the reaction chamber (13) and in turn connected to the vacuum pump (12) so as to separate the reactant liquid exiting the reaction chamber (13) from the air; and the residue tank being connected to the reaction chamber (13) below the pervaporation membrane assembly (14) so as to collect a residual solution after reaction.

7. A method for fixing an artificial bioprosthetic valve by membrane pervaporation using the device according to any one of claims 1-6, **characterized by** comprising the steps of:
fixing one end of the artificial bioprosthetic valve to the fixation clamp (9) of the device and leaving the other end freely suspended; activating the vacuum pump (12);
activating the pervaporation membrane assembly (14) to allow the vaporized reactant liquid to enter the reaction chamber (13) and permeate through the pervaporation membrane assembly (14) to the artificial bioprosthetic valve; and
letting the artificial bioprosthetic valve react in the reaction chamber (13) for a period of time,
wherein the reactant liquid comprises a glutaraldehyde solution.

8. The method for fixing an artificial bioprosthetic valve by membrane pervaporation according to claim 7, **characterized by** further comprising the step of: activating the stirring motor (2) to drive the stirring rod (3) and the stirring blade (4) so as to stir the reactant liquid in the storage tank (15).

9. The method for fixing an artificial bioprosthetic valve by membrane pervaporation according to claim 7, **characterized by** further comprising the step of: activating the rotating motor (6), by which the rotation speed of the rotating disc (8) is adjusted.

10. The method for fixing an artificial bioprosthetic valve by membrane pervaporation according to any one of claims 7-9, **characterized in that** the method is carried out within the following ranges of process parameters:
the permeation rate of the membrane assembly: 20∼180 g/(m² · hour);
the pressure of the reaction chamber (13): 0∼5000 Pa;
the concentration of the glutaraldehyde solution: 0.5∼50%;
the rotation speed of the stirring rod (3): 0∼200 rpm; and
the rotation speed of the rotating disc (8): 0∼200 rpm.

## Patentansprüche

1. Vorrichtung zum Fixieren einer künstlichen bioprothetischen Klappe durch Membranpervaporation, umfassend einen Speicherbehälter (15) zum Speichern einer Reaktantenflüssigkeit, wobei die Reaktantenflüssigkeit aus einer Gruppe ausgewählt ist, die aus einer Glutaraldehydlösung, einer Formaldehydlösung und einer Acetaldehydlösung besteht, eine Befestigungsklemme (9) zum Befestigen der künstlichen bioprothetischen Klappe, eine Reaktionskammer (13) und eine Vakuumpumpe (12), wobei die Befestigungsklemme (9) im Inneren der Reaktionskammer (13) angeordnet ist, **dadurch gekennzeichnet, dass** die Vorrichtung eine Pervaporationsmembran-Anordnung (14) umfasst, die im Inneren der Reaktionskammer (13) stromaufwärts von der Befestigungsklemme (9) angeordnet ist, wobei der Speicherbehälter (15) stromaufwärts von der Reaktionskammer (13) mit der Reaktionskammer (13) verbunden ist und die Vakuumpumpe (12) stromabwärts von der Reaktionskammer (13) mit der Reaktionskammer (13) verbunden ist.

2. Vorrichtung zum Fixieren einer künstlichen bioprothetischen Klappe durch Membranpervaporation nach Anspruch 1, **dadurch gekennzeichnet, dass** die Pervaporationsmembran-Anordnung (14) eines oder mehreres aus einer flachen Pervaporationsmembran-Anordnung (14), einer Hohlfaser-Pervaporationsmembran-Anordnung (14) und einer rollenförmigen Pervaporationsmembran-Anordnung (14) umfasst.

3. Vorrichtung zum Fixieren einer künstlichen bioprothetischen Klappe durch Membranpervaporation nach Anspruch 1 oder 2, **dadurch gekennzeichnet, dass** die Vorrichtung ferner Folgendes umfasst: einen Rührmotor (2), einen Rührstab (3) und ein Rührblatt (4), wobei der Rührstab (3) an einem Endabschnitt des Rührmotors (2) befestigt ist und das Rührblatt (4) an dem Rührstab (3) befestigt ist, wodurch der Rührstab (3) und das Rührblatt (4) durch den Rührmotor (2) angetrieben werden, um die Reaktantenflüssigkeit in dem Speicherbehälter (15) zu rühren.

4. Vorrichtung zum Fixieren einer künstlichen bioprothetischen Klappe durch Membranpervaporation nach Anspruch 1 oder 2, **dadurch gekennzeichnet, dass** die Vorrichtung ferner eine Drehscheibe (8) und einen Drehmotor (6) umfasst, wobei die Drehscheibe (8) an einem Endabschnitt des Drehmotors (6) befestigt ist und im Inneren der Reaktionskammer (13) angeordnet ist; und wobei die Befestigungsklemme (9) an der Drehscheibe (8) befestigt ist, wodurch die Drehgeschwindigkeit der Drehscheibe (8) durch den Drehmotor (6) eingestellt wird.

5. Vorrichtung zum Fixieren einer künstlichen bioprothetischen Klappe durch Membranpervaporation nach Anspruch 1 oder 2, **dadurch gekennzeichnet, dass** die Vorrichtung ferner Heizmodul-Anordnungen umfasst, wobei die Heizmodul-Anordnungen am Umfang des Speicherbehälters (15) bzw. der Reaktionskammer (13) befestigt sind, um die Verdampfung der Reaktantenflüssigkeit zu erleichtern.

6. Vorrichtung zum Fixieren einer künstlichen bioprothetischen Klappe durch Membranpervaporation nach Anspruch 1 oder 2, **dadurch gekennzeichnet, dass** die Vorrichtung ferner einen Gas-Flüssigkeits-Abscheider und einen Rückstandsbehälter umfasst, wobei der Gas-Flüssigkeits-Abscheider stromabwärts von der Reaktionskammer (13) mit der Reaktionskammer (13) verbunden ist und seinerseits mit der Vakuumpumpe (12) verbunden ist, um die aus der Reaktionskammer (13) austretende Reaktantenflüssigkeit von der Luft zu trennen; und wobei der Rückstandsbehälter unter der Pervaporationsmembran-Anordnung (14) mit der Reaktionskammer (13) verbunden ist, um nach der Reaktion eine Rückstandslösung aufzufangen.

7. Verfahren zum Fixieren einer künstlichen bioprothetischen Klappe durch Membranpervaporation unter Verwendung der Vorrichtung nach einem der Ansprüche 1 bis 6, **dadurch gekennzeichnet, dass** es die folgenden Schritte umfasst:
Befestigen eines Endes der künstlichen bioprothetischen Klappe an der Befestigungsklemme (9) der Vorrichtung und Belassen des anderen Endes in frei hängendem Zustand; Aktivieren der Vakuumpumpe (12);
Aktivieren der Pervaporationsmembran-Anordnung (14), um es der verdampften Reaktantenflüssigkeit zu ermöglichen, in die Reaktionskammer (13) einzutreten und die Pervaporationsmembran-Anordnung (14) in Richtung auf die künstliche bioprothetische Klappe zu durchdringen; und
Reagierenlassen der künstlichen bioprothetischen Klappe in der Reaktionskammer (13) während eines Zeitraums,
wobei die Reaktantenflüssigkeit eine Glutaraldehydlösung umfasst.

8. Verfahren zum Fixieren einer künstlichen bioprothetischen Klappe durch Membranpervaporation nach Anspruch 7, **dadurch gekennzeichnet, dass** es ferner den folgenden Schritt umfasst: Aktivieren des Rührmotors (2), um den Rührstab (3) und das Rührblatt (4) anzutreiben, um die Reaktantenflüssigkeit in dem Speicherbehälter (15) zu rühren.

9. Verfahren zum Fixieren einer künstlichen bioprothetischen Klappe durch Membranpervaporation nach Anspruch 7, **dadurch gekennzeichnet, dass** es ferner den folgenden Schritt umfasst: Aktivieren des Drehmotors (6), durch den die Drehgeschwindigkeit der Drehscheibe (8) eingestellt wird.

10. Verfahren zum Fixieren einer künstlichen bioprothetischen Klappe durch Membranpervaporation nach einem der Ansprüche 7 bis 9, **dadurch gekennzeichnet, dass** das Verfahren innerhalb der folgenden Prozessparameterbereiche durchgeführt wird:
Permeationsrate der Membrananordnung: 20 - 180 g/(m²·Stunde);
Druck der Reaktionskammer (13): 0 bis 5000 Pa;
Konzentration der Glutaraldehydlösung: 0,5 bis 50%;
Drehgeschwindigkeit des Rührstabs (3): 0 bis 200 U.p.M.; und
Drehgeschwindigkeit der Drehscheibe (8): 0 bis 200 U.p.M.

## Revendications

1. Dispositif pour fixer une valve bioprothétique artificielle par pervaporation sur membrane, comprenant un réservoir de stockage (15) pour stocker un réactif liquide, lequel réactif liquide est choisi parmi un groupe comprenant une solution de glutaraldéhyde, une solution de formaldéhyde et une solution d'acétaldéhyde, une pince de fixation (9) pour fixer la valve bioprothétique artificielle, une chambre de réaction (13) et une pompe à vide (12), la pince de fixation (9) étant positionnée à l'intérieur de la chambre de réaction (13), **caractérisé en ce que** le dispositif comprend un assemblage de membrane de pervaporation (14) positionné à l'intérieur de la chambre de réaction (13) en amont de la pince de fixation (9), le réservoir de stockage (15) étant raccordé à la chambre de réaction (13) en amont de la chambre de réaction (13) et la pompe à vide (12) étant raccordée à la chambre de réaction (13) en aval de la chambre de réaction (13).

2. Dispositif pour fixer une valve bioprothétique artificielle par pervaporation sur membrane selon la revendication 1, **caractérisé en ce que** l'assemblage de membrane de pervaporation (14) comprend un ou plusieurs assemblages parmi un assemblage de membrane de pervaporation (14) à plat, un assemblage de membrane de pervaporation (14) à fibres creuses et un assemblage de membrane de pervaporation (14) à enroulement.

3. Dispositif pour fixer une valve bioprothétique artificielle par pervaporation sur membrane selon la revendication 1 ou 2, **caractérisé en ce que** le dispositif comprend en outre : un moteur d'agitation (2), une tige d'agitation (3) et une pale d'agitation (4), la tige d'agitation (3) étant fixée à une partie d'extrémité du moteur d'agitation (2) et la pale d'agitation (4) étant fixée à la tige d'agitation (3), la tige d'agitation (3) et la pale d'agitation (4) étant ainsi entraînées par le moteur d'agitation (2) pour agiter le réactif liquide dans le réservoir de stockage (15).

4. Dispositif pour fixer une valve bioprothétique artificielle par pervaporation sur membrane selon la revendication 1 ou 2, **caractérisé en ce que** le dispositif comprend en outre un disque rotatif (8) et un moteur rotatif (6), le disque rotatif (8) étant fixé à une partie d'extrémité du moteur rotatif (6) et situé à l'intérieur de la chambre de réaction (13), et la pince de fixation (9) étant fixée au disque rotatif (8), de sorte que la vitesse de rotation du disque rotatif (8) est ajustée par le moteur rotatif (6).

5. Dispositif pour fixer une valve bioprothétique artificielle par pervaporation sur membrane selon la revendication 1 ou 2, **caractérisé en ce que** le dispositif comprend en outre des assemblages de module chauffant qui sont fixés sur la périphérie du réservoir de stockage (15) et de la chambre de réaction (13), respectivement, pour faciliter l'évaporation du réactif liquide.

6. Dispositif pour fixer une valve bioprothétique artificielle par pervaporation sur membrane selon la revendication 1 ou 2, **caractérisé en ce que** le dispositif comprend en outre un séparateur gaz-liquide et un réservoir à résidus, le séparateur gaz-liquide étant raccordé à la chambre de réaction (13) en aval de la chambre de réaction (13) et raccordé à sont tour à la pompe à vide (12) afin de séparer le réactif liquide sortant de la chambre de réaction (13) de l'air, et le réservoir à résidus étant raccordé à la chambre de réaction (13) en dessous de l'assemblage de membrane de pervaporation (14) afin de recueillir une solution résiduelle après la réaction.

7. Procédé pour fixer une valve bioprothétique artificielle par pervaporation sur membrane à l'aide du dispositif selon l'une quelconque des revendications 1 à 6, **caractérisé en ce qu'**il comprend les étapes de :
fixation d'une extrémité de la valve bioprothétique artificielle à la pince de fixation (9) du dispositif en laissant l'autre extrémité suspendue librement ;
activation de la pompe à vide (12) ;
activation de l'assemblage de membrane de pervaporation (14) afin de permettre au réactif liquide vaporisé d'entrer dans la chambre de réaction (13) et d'imprégner l'assemblage de membrane de pervaporation (14) en le traversant jusqu'à la valve bioprothétique artificielle ; et
maintien de la valve bioprothétique artificielle en réaction dans la chambre de réaction (13) pendant un certain temps,
le réactif liquide contenant une solution de glutaraldéhyde.

8. Procédé pour fixer une valve bioprothétique artificielle par pervaporation sur membrane selon la revendication 7, **caractérisé en ce qu'**il comprend en outre l'étape d'activation du moteur d'agitation (2) pour entraîner la tige d'agitation (3) et la pale d'agitation (4) afin d'agiter le réactif liquide dans le réservoir de stockage (15).

9. Procédé pour fixer une valve bioprothétique artificielle par pervaporation sur membrane selon la revendication 7, **caractérisé en ce qu'**il comprend en outre l'étape d'activation du moteur rotatif (6) de façon à régler la vitesse de rotation du disque rotatif (8).

10. Procédé pour fixer une valve bioprothétique artificielle par pervaporation sur membrane selon l'une quelconque des revendications 7 à 9, **caractérisé en ce que** le procédé est exécuté dans les plages suivantes de paramètres de process :
taux de perméation de l'assemblage de membrane : de 20 à 180 g/m²/heure ;
pression de la chambre de réaction (13) : de 0 à 5000 Pa ;
concentration de la solution de glutaraldéhyde : de 0,5 à 50 % ;
vitesse de rotation de la tige d'agitation (3) : de 0 à 200 tr./min et
vitesse de rotation du disque rotatif (8) : de 0 à 200 tr./min.
